# EUROPEAN PATENT APPLICATION

(11) **EP 2 626 365 A1**
(43) Date of publication of application: **14.08.2013**
(21) Application number: 12154831.7
(22) Date of filing: 10.02.2012
(51) Int. Cl.: C07K 14/44, G01N 33/569

(54) **Compounds and methods for the diagnosis of babesia canis canis infection**

(71) Applicant: MegaCor Diagnostik GmbH, 6912 Hörbranz (AT)
(72) Inventor: Shinuo, Cao, Obihiro, Hokkaido 080-0838 (JP); Xuan, Xuenan, Obihiro, Hokkaido 080-0834 (JP); Igarashi, Ikuo, Obihiro, Hokkaido 080-2474 (JP)
(74) Representative: Grünecker, Kinkeldey, Stockmair & Schwanhäusser

(57) **Abstract**

The present invention relates to compounds and methods for the diagnosis of a *Babesia canis canis* infection. The compounds provided are polypeptides comprising at least an antigenic fragment of a *Babesia canis canis* antigen, antibodies that specifically bind to the polypeptides, and nucleic acids encoding the polypeptides. Further provided are compositions, vaccines and diagnostic kits comprising the compounds of the present invention. The compounds of the present invention can be used in methods of detecting the presence of antibodies against *Babesia canis canis* in a biological sample and in methods of diagnosing a *Babesia canis canis* infection.

SEQUENCE LISTING

<110>  MegaCor Diagnostik GmbH

<120>  Development of Serological Diagnostic Methods against Babesia
       Canis Canis Infection

<130>  EP76513HVSE

<140>  not yet assigned
<141>  herewith

<160>  4

<170>  PatentIn version 3.3

<210>  1
<211>  321
<212>  PRT
<213>  Babesia canis canis

<220>
<223>  BccP33

<400>  1

Met Met Leu Leu Phe Ala Leu Ser Thr Leu Val Thr Phe Ala Phe Cys
1               5                   10                  15

Asp Gly Glu Asn Thr Ile Leu Leu Ser Asn Val Glu Phe His Thr Pro
            20                  25                  30

Val Ser Ser Val Lys Leu Leu Lys Glu Tyr Ser Ser Asn Gln Glu Ser
        35                  40                  45

Met Ala Val Ile Met Met Leu Thr Glu Met Pro Asn Thr Ser Gly Lys
    50                  55                  60

Leu Thr Asp Gly Lys Val His Val Ala Asn Asp Asn Val Lys Cys Ala
65                  70                  75                  80

Asp Leu Ala Leu Ala Tyr Gln Glu Leu Lys Lys Ala Gly Lys Val Thr
                85                  90                  95

Ser Trp Ser Pro Thr Asp Asp Asn Asp Lys Val Val Pro His Gly Ile
            100                 105                 110

Trp Phe Ile Glu Gly Val Tyr Glu Thr Asp Lys Met Phe Glu Val Tyr
        115                 120                 125

Lys Thr Leu Thr Asp Pro Glu Asp Pro Ser Glu Val Thr Arg Leu Thr
    130                 135                 140

Thr Val Ser Gly Ala Ser Gly Ser Ala Gln Ser Gln Pro Gly Val Thr
145                 150                 155                 160

Thr Gly Gly Val Ser Gly Ser Ala Ala Ser Ala Ser Gly Ser Ser Gly
                165                 170                 175

Ser Thr Thr Ser His Ser Thr Thr Val Thr Thr Thr Ser Thr Ser Thr
            180                 185                 190

Val Ser Thr Pro Ser Ser Gly Thr Ser Thr Ser Thr Ser Thr Asp Arg
        195                 200                 205

Ser Ser Val Leu Gly Thr Gln Thr Ser Tyr Ser Ala Glu Ser Ser Val
    210                 215                 220

His Lys Ser Ser Val Val Ala Ser Thr Gln Ser Thr Thr Ser Glu Asn
225                 230                 235                 240

Ala Glu Ser Val Glu Lys Gln Ser Lys Ala Ala Val Gln Glu Pro Lys
                245                 250                 255

Asn Val Leu Met Ile Leu Thr Lys Cys Asp Leu Asn Ala Lys Val Thr
            260                 265                 270

Glu Glu Gln Ile Arg Ser Gln Gly Asn Pro Glu Ser Asn Gly Ser Ser
        275                 280                 285

Ser Glu Pro Thr Ala Ala Ser Pro Lys Leu Thr Thr Ala Ala Ser Gly
    290                 295                 300

Phe Thr Ala Ala Ile Thr Pro Leu Phe Met Val Pro Leu Met Phe Phe
305                 310                 315                 320

Ala

<210>  2
<211>  1255
<212>  DNA
<213>  Babesia canis canis

<220>
<223>  BccP33

<400>  2
ggcacgaggt tttaatgttt tggtagtttc acaattctag aaggtcaaga tgatgctgct      60

cttcgccttg tctactcttg tcaccttcgc cttctgcgat ggtgaaaaca ctatactttt     120

atccaatgta gaattccata ctccagtatc cagtgtaaag ctgcttaaag aatacagtag     180

caatcaggaa tcaatggccg ttattatgat gctaaccgaa atgccaaaca catcaggaaa     240

gcttaccgat ggcaaagttc atgtggccaa tgataacgtt aaatgtgctg atttggctct     300

ggcttatcaa gaactcaaaa aggccggcaa ggtgacatca tggagtccaa ctgatgacaa     360

cgataaggtg gtacctcatg gaatctggtt catagagggc gtctatgaga ctgataagat     420

gttcgaggtt tacaagaccc tgaccgatcc tgaggaccca agcgaagtta ctcgcttgac     480

aactgtttct ggtgcttccg gatctgctca aagccagcct ggtgttacta ctggtggtgt     540

ctccggtagc gctgctagtg cctctggttc ctctggttcc actacttctc attctactac 600

cgttactacc acctccacta gtaccgtttc tactccctct tctgggactt ctacttctac 660

ttctactgac cggtcatctg tgcttggcac ccaaacatcc tacagtgcag aatctagcgt 720

tcataaaagc tctgtcgttg cttccactca gagtaccact tctgagaatg ccgaatctgt 780

cgaaaagcaa agcaaagctg cggttcagga acctaagaat gttttgatga ttctgaccaa 840

gtgtgatctt aacgccaaag ttaccgagga acagataaga agccaaggaa acccagaaag 900

caatgggtct tcttctgaac ccactgctgc ttctcctaaa cttactaccg ctgcttctgg 960

attcactgcc gccattactc ccttgttcat ggtcccactc atgtttttcg cctaataaga 1020

cgtaagaaat agaaccaaac atacctccga agctgtctgg caacacgttt gacaagaatt 1080

cttaatctag aaacgaaatg aggattggct tcttattctg gaacaccatt tgaattaaga 1140

ttgtagtggc aaacaccgcc atttataagg gaaaagctcc ggtattgttt ctttatagac 1200

tagaaaatat acatcattca atttaatggc tataaacaaa aaaaaaaaaa aaaaa 1255

<210> 3
<211> 417
<212> PRT
<213> Babesia canis canis

<220>
<223> BccSA1

<400> 3

Ala Arg Gly Leu Val Pro Asn Ser Ala Arg Gly Phe Tyr Pro Leu Asn
1               5                   10                  15

Leu Leu Lys Thr His Pro His Asn Phe Ile Leu Ile Phe Gln Leu Leu
            20                  25                  30

Ala Thr Met Gly Asn Ala Gln Ser Thr Ser Ser Gln Glu Asn Ser Arg
        35                  40                  45

Asp Gly Leu Arg Glu Val Leu Glu Tyr Thr Asn Gln Leu His Asn Asn
    50                  55                  60

Tyr Gly Ser Ala Val Arg Lys Val Thr Asp Lys Leu Lys Asn Glu Ile
65                  70                  75                  80

Asp Val Tyr Cys Lys Ser Thr Asp Asp Lys Gly Tyr Tyr Phe Ala Asn
                85                  90                  95

Gly Ser Phe Gly Tyr Phe Arg Lys Ala Leu Asn Asp Ser Phe Asn Phe
            100                 105                 110

Arg Phe Gln Leu Leu Ser Asn Tyr Asn Asp Tyr Arg Lys Tyr Lys Thr
        115                 120                 125

Arg Phe Gln Asp Thr Asp Asp Glu Ala Glu Lys His Val Lys Tyr Leu
    130                 135                 140

Lys Glu Asn Leu Phe Asp Leu Phe Gly Thr Leu Ser Tyr Met Tyr Phe
145                 150                 155                 160

Gln Cys Ser His Lys Cys Gln Lys Tyr Asn Gly Gly Lys Trp Glu Glu
                165                 170                 175

Gln Ser Met Asn Gln Ser Gly Ser Glu Val Ser Lys Trp Leu Met Gly
            180                 185                 190

Ser Asn Ser Ala Ala Thr Asp Ser Val His Phe Leu Gly Arg Asp Phe
        195                 200                 205

Ser Thr Ser Glu Leu Thr Asn Ile Lys Gly Lys Glu Leu Ala Asp Lys
    210                 215                 220

Asp Arg Ala Ser Leu Ser Asp Leu Ile Lys Tyr Ser Gly Arg Gly Asn
225                 230                 235                 240

Leu Gln His Ala Leu Phe Trp Met Leu Phe Ile Gly Pro Trp Val Asp
                245                 250                 255

Gly Lys Thr Gly His Ala Ile Leu Phe Leu Ile Gln Phe Ile Glu Leu
            260                 265                 270

Thr Glu Tyr Asp Tyr Phe Lys Glu Asn Ser Lys Lys Lys Tyr Asp Tyr
        275                 280                 285

Lys Lys Leu Val Thr Pro Cys Lys Asn Leu Lys Thr Pro Leu Gly Asp
    290                 295                 300

Val Ser Arg Lys Val Val Pro Val Gly Lys Asn Pro Asn Val Lys Tyr
305                 310                 315                 320

Arg Asp Leu Leu Ser Gly Glu Phe Phe Asp Ala Tyr Val Asp Trp Leu
                325                 330                 335

Glu Lys Asn Ile Asp Asn Leu Ile Lys Ser Leu Glu Asp Met Ser Lys
            340                 345                 350

Asp Cys Asn Asn Trp Thr Tyr Asp Lys Leu Asn Ser Ala Ser Ser Tyr
        355                 360                 365

Gly Pro Phe Pro Trp Gly Cys Val Phe Lys Asp Glu Asp Trp His Thr
    370                 375                 380

Ser Gly Arg Leu Pro Lys Leu Pro Glu Asp Val Lys Lys Ala Thr Asp
385                 390                 395                 400

Ala Leu Lys Lys Leu Lys Glu Ala Ile Glu Glu Met Lys Lys Glu Lys
                405                 410                 415

Lys

<210>  4
<211>  1460
<212>  DNA
<213>  Babesia canis canis

<220>
<223>  BccSA1

<400>  4
gcacgaggcc tcgtgccgaa ttcggcacga gggttctatc cattaaacct cctgaaaact      60

catccacata attttatttt aatttttcaa ttattagcaa caatgggtaa cgctcaatca     120

acaagcagcc aggaaaactc acgggatggc ttacgggaag tcttggaata tacgaaccag     180

ctgcacaaca actacggttc tgcagtaagg aaggttacag acaagcttaa gaatgagatt     240

gacgtatact gcaaatccac tgatgataag ggttactact tcgccaatgg ttctttcgga     300

tatttccgca aggccctcaa cgattccttc aactttaggt tccagttgct ctccaactac     360

aacgattaca ggaagtataa gacgcgcttc caggacaccg atgatgaggc cgaaaagcac     420

gttaagtacc tcaaagaaaa ccttttcgat ctattcggta ccttgtccta catgtatttc     480

cagtgctctc acaaatgcca gaaatacaac ggagggaaat gggaagaaca aagtatgaac     540

caaagtggta gtgaggtgag caaatggctt atggggagta attctgctgc cactgactcc     600

gttcattttc tggggaggga cttctcaact tcagagttaa ccaacatcaa aggcaaagag     660

ctggccgaca aggatcgcgc tagcctatct gatttaatta aatacagtgg cagaggtaac     720

cttcagcatg ccctgttctg gatgctgttc attggaccat gggtggatgg aaaaactggt     780

catgccatac tgttcttgat ccaatttatc gaattaacag aatatgatta cttcaaagag     840

aatagcaaga agaagtacga ttacaaaaag ctggttaccc cttgcaagaa tctgaagacc     900

cctcttggtg acgtatcaag gaaagtagta cctgtaggaa agaaccccaa cgttaaatac     960

agagacctcc tatccggcga atttttcgat gcatatgttg attggttgga gaagaacatt    1020

gacaacctta ttaaatcttt ggaggacatg tctaaagact gcaacaattg gacttacgat    1080

aaactaaaca gcgcgtcttc ctatggtcct ttcccatggg gatgtgtttt caaagatgag    1140

gattggcaca ccagtggtag gctgccaaag ttgcctgaag atgtcaagaa agccactgat    1200

gccttaaaaa aacttaaaga ggctattgaa gaaatgaaga aagagaaaaa gtaagaatga    1260

atcaactagt tttaatttgt gacttgccat cctactaaca tggaattcgt ttagaaaatg    1320

ttgcttctgg gtggtgaaga cgcaacattc atgtcagcgt aaatttccga tgcgtatttg    1380

ttgccatgta aaatacgcat ggtcactcaa cacatactga ttttaatctt attataacat    1440

ttaaaaaaaa aaaaaaaaaa                                                1460

## Description

### FIELD OF THE INVENTION

The present invention relates to compounds and methods for the diagnosis of a *Babesia canis canis* infection. The compounds provided are polypeptides comprising at least an antigenic fragment of a *Babesia canis canis* antigen, antibodies that specifically bind to the polypeptides, and nucleic acids encoding the polypeptides. Further provided are compositions, vaccines and diagnostic kits comprising the compounds of the present invention. The compounds of the present invention can be used in methods of detecting the presence of antibodies against *Babesia canis canis* in a biological sample and in methods of diagnosing a *Babesia canis canis* infection.

### BACKGROUND OF THE INVENTION

*Babesia canis canis (B. canis canis)* is a tick-borne hemoprotozoan parasite that causes piroplasmosis in dogs, which is transmitted by the hard tick *Dermacentor reticulatus.* The disease is characterized by varying degrees of remittent fever, thrombocytopenia, hemolytic anemia, hemoglobinuria and sometimes causes death. Currently, *B. canis canis* infection is mainly endemic in Europe and West Asia. This disease has become an important emerging disease and is of serious clinical concern. Therefore, the detection of dogs that are carriers or that have a chronic form of this disease is very important. Generally, the diagnosis of acute babesiosis is carried out by detection of intraerythrocytic *Babesia* organisms by microscopy of a Giemsa-stained thin blood smear. However, the detection of parasites in most atypical or chronic cases may become difficult due to scarce parasitemia.

Polymerase chain reaction (PCR) is an alternative test, with good sensitivity and specificity, and is able to detect early or carrier infections, but the test requires specialized laboratory equipment and facilities and well-trained laboratory personnel. On the other hand, immunofluorescent antibody test (IFAT) has proved useful in the detection of sub-clinical cases and field surveys. However, IFAT is not suitable for use in a large number of serum samples for epidemiological surveys. Moreover, the results of IFAT may be influenced by the subjective judgment of the operator. Alternatively, an enzyme-linked immunosorbent assay (ELISA) is quite sensitive and is appropriate for large-scale testing especially in field surveys. However, so far there is no recombinant antigen available for serological diagnostic methods for *B. canis canis.* Moreover, the poor quality of the antigens and sometimes cross-reactions between the *Babesia* species and closely related Apicomplexan parasites has limited their application.

Thus, there remains a need in the art for sensitive and specific serological assays for the detection of antibodies against *Babesia canis canis.*

### SUMMARY OF THE INVENTION

The present invention generally relates to compounds, compositions, kits and methods for detecting antibodies against *Babesia canis canis* and specifically, for diagnosing a *Babesia canis canis* infection in dogs.

Main aspects of the invention are:
1. A nucleic acid molecule selected from the group consisting of:
   (a) a nucleic acid comprising a polynucleotide encoding a polypeptide having the amino acid sequence of SEQ ID NO: 1;
   (b) a nucleic acid comprising a polynucleotide encoding a polypeptide having the amino acid sequence of SEQ ID NO: 3;
   (c) a nucleic acid comprising the nucleotide sequence of SEQ ID NO: 2;
   (d) a nucleic acid comprising the nucleotide sequence of SEQ ID NO: 4;
   (e) a nucleic acid having the nucleotide sequence of SEQ ID NO: 2;
   (f) a nucleic acid having the nucleotide sequence of SEQ ID NO: 4;
   (g) a nucleic acid encoding a fragment of a polypeptide having the amino acid sequence of SEQ ID NO: 1 or SEQ ID NO: 3, wherein said polypeptide fragment is an antigenic fragment specific for *Babesia canis canis*;
   (h) a nucleic acid comprising a polynucleotide which is at least 80% identical to the nucleotide sequence of SEQ ID NO: 2, wherein said polynucleotide encodes an antigen specific for *Babesia canis canis;*
   (i) a nucleic acid comprising a polynucleotide which is at least 80% identical to the nucleotide sequence of SEQ ID NO: 4, wherein said polynucleotide encodes an antigen specific for *Babesia canis canis;*
   (j) a nucleic acid which is at least 80% identical to the nucleic acid of (g), wherein the nucleic acid encodes a fragment of a polypeptide having the amino acid sequence of SEQ ID NO: 1 or SEQ ID NO: 3 and wherein said fragment is an antigenic fragment specific for *Babesia canis canis;* and
   (k) a nucleic acid that is the complement of any of (a) to (j).
2. A polypeptide selected from the group consisting of:
   (a) a polypeptide encoded by a nucleic acid molecule of any of claim 1 (a) to (j);
   (b) a polypeptide comprising the amino acid sequence of SEQ ID NO: 1;
   (c) a polypeptide comprising the amino acid sequence of SEQ ID NO: 3;
   (d) a polypeptide having the amino acid sequence of SEQ ID NO: 1;
   (e) a polypeptide having the amino acid sequence of SEQ ID NO: 3;
   (f) a polypeptide having an amino acid sequence which is at least 80 % identical to the amino acid sequence of SEQ ID NO: 1, wherein said polypeptide is an antigen specific for *Babesia canis canis;*
   (g) a polypeptide having an amino acid sequence which is at least 80 % identical to the amino acid sequence of SEQ ID NO: 3, wherein said polypeptide is an antigen specific for *Babesia canis canis;*
   (h) a polypeptide comprising the polypeptide of (f) or (g); and
   (i) a fragment of any of (a) to (g), wherein said polypeptide fragment is an antigenic fragment specific for *Babesia canis canis.*
3. A vector comprising a nucleic acid of item 1.
4. A host cell comprising a nucleic acid of item 1 or the vector of item 3.
5. An antibody, or antigen-binding fragment thereof, that specifically binds to a polypeptide of item 2.
6. A composition comprising at least one component selected from the group consisting of:
   (i) a nucleic acid of item 1;
   (ii) a polypeptide of item 2;
   (iii) the vector of item 3; and
   (iv) the antibody or antigen-binding fragment thereof of item 5.
7. A vaccine comprising at least one component selected from the group consisting of:
   (i) a nucleic acid of item 1;
   (ii) a polypeptide of item 2; and
   (iii) the vector of item 3.
8. A diagnostic kit comprising at least one component selected from the group consisting of:
   (i) a nucleic acid of item 1;
   (ii) a polypeptide of item 2;
   (iii) the vector of item 3; and
   (iv) the antibody of item 5.
9. A method of detecting antibodies against *Babesia canis canis* in a biological sample, comprising
   (i) contacting a polypeptide of item 2 with the biological sample, and
   (ii) detecting the presence of antibodies that bind to the polypeptide or antigenic fragment in the sample.
10. Method of diagnosing a *Babesia canis canis* infection in a dog, comprising
   (i) contacting a polypeptide of item 2 with a biological sample obtained from the dog;
   (ii) detecting the presence of antibodies that bind to the polypeptide or antigenic fragment in the sample, wherein the presence of said detected antibodies is indicative of a *Babesia canis canis* infection.
11. The method of item 9 or 10, wherein the detection is carried out by ELISA.
12. Use of a nucleic acid of item 1, or a polypeptide of item 2, or the antibody of item 5, in a method of detecting *Babesia canis canis.*
13. Use of a polypeptide of item 2 in a method of detecting antibodies against *Babesia canis canis.*
14. Use of a nucleic acid of item 1, or a polypeptide of item 2, or the antibody of item 5, in a method of diagnosing a *Babesia canis canis* infection.
15. Use of a nucleic acid of item 1, or a polypeptide of item 2, or the vector of item 3, or the antibody of item 5, for preparing a vaccine.

### BRIEF DESCRIPTION OF THE DRAWINGS

Fig. 1 shows the nucleotide sequence of the cDNA coding for *Bcc*P33 and the amino acid sequence derived from the nucleotide sequence. The signal peptide is underlined.
Fig. 2 shows the structure of the genomic *Bcc*P33 gene. White bold lines indicate exons, and black rectangles indicated introns. The numeric numbers indicate the number of nucleotides.
Fig. 3 shows the nucleotide sequence of the cDNA coding for *Bcc*SA1 and the amino acid sequence derived from the cDNA. The signal peptide is underlined.
Fig. 4 shows the result of the software analysis of hydrophilicity, surface probability and antigenicity of *Bcc*P33 and *Bcc*SA1.

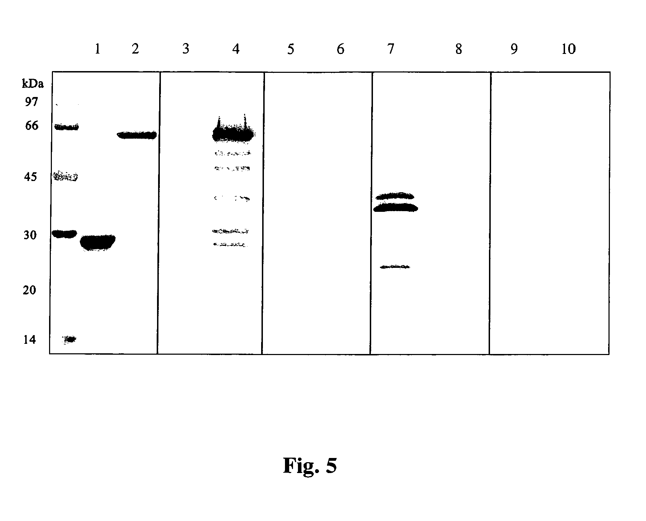

Fig. 5 shows the results of SDS-PAGE and Western blot analysis of recombinant and native *Bcc*P33. Lanes 1 and 2, the purified rGST and r*Bcc*P33 fused with GST were stained by amide black. Lanes 3 and 4, the purified rGST and r*Bcc*P33 fused with GST were incubated with a serum from a dog infected with *B. canis canis*; lanes 5 and 6, the purified rGST and r*Bcc*P33 fused with GST were incubated with a serum from a healthy dog; lanes 7 and 8, *B. canis canis*-infected dog erythrocyte lysate and normal dog erythrocyte lysate were incubated with mouse polyclonal antibody against r*Bcc*P33; lanes 9 and 10, *B. canis canis*-infected dog erythrocyte lysate and normal dog erythrocyte lysate were incubated with a normal mouse serum
Fig. 7 shows the results of the detection of circulating *Bcc*SA1 in plasma from a dog experimentally infected with *B. canis canis* by Western blot. Lane 1: the purified r*Bcc*SA1 was stained with amide black. Lanes 2 and 3: *B. canis canis*-infected dog plasma and normal dog plasma were incubated with mouse polyclonal antibody against r*Bcc*SA1.
Fig. 8 shows that there is no cross-reactivity of r*Bcc*P33 with closely related parasite-infected dog sera as determined by ELISA. Lane 1: sera from *B. canis canis*-infected dogs (n=12); lane 2, SPF dog sera (n=28); lane 3, sera from *B. canis rossi*-infected dogs (n=3); lane 4, sera from *B. canis vogeli*-infected dogs (n=3); lane 5, sera from *B. gibsoni*-infected dogs (n=3); lane 6, sera from *N. caninum*-infected dogs (n=3).
Fig. 9 shows that there is no cross-reactivity of r*Bcc*SA1 with closely related parasite-infected dog sera as determined by ELISA. Lane 1, sera from *B. canis canis*-infected dogs (n=11); lane 2: SPF dog sera (n=28); lane 3: sera from *B. canis rossi*-infected dogs (n=3); lane 4: sera from *B. canis vogeli*-infected dogs (n=3); lane 5: sera from *B. gibsoni*-infected dogs (n=3); lane 6: sera from *N. caninum*-infected dogs (n=3).
Fig. 10 shows the detection of antibodies against *Bcc*P33 and *Bcc*SA1 in a dog experimentally infected with *B. canis canis* by the ELISA using r*Bcc*P33 and r*Bcc*SA1.

### DETAILED DESCRIPTION OF THE INVENTION

As noted above, the present invention generally relates to compounds, compositions, kits and methods for detecting antibodies against *Babesia canis canis* and for diagnosing a *Babesia canis canis* infection in dogs.

In one aspect, the present invention provides nucleic acids comprising a polynucleotide encoding a polypeptide having an amino acid sequence selected from the group consisting of the amino acid set forth in SEQ ID NO: 1 and the amino acid set forth in SEQ ID NO: 3. In a preferred embodiment, the nucleic acid comprises a nucleotide sequence selected from the group consisting of the nucleotide sequence set forth in SEQ ID NO: 2 and the nucleotide sequence set forth in SEQ ID NO: 4, or a fragment thereof. Preferably, the nucleic acid has the nucleotide sequence set forth in SEQ ID NO: 2 or SEQ ID NO: 4, or is a fragment of the nucleotide sequence set forth in SEQ ID NO: 2 or SEQ ID NO: 4.

Also provided are nucleic acids encoding a fragment of a polypeptide having the amino acid sequence of SEQ ID NO: 1 or SEQ ID NO: 3, wherein said polypeptide fragment is an antigenic fragment specific for *Babesia canis canis*, including nucleic acids which are at least 80% identical to said fragment-encoding nucleic acids. *Babesia canis canis* specificity may be determined e.g. by using the ELISA assay described herein or any other suitable method known in the art.

Fragments of the present invention (including fragments of the nucleotide sequences of SEQ ID NO: 2 and SEQ ID NO: 4) may be combined with other DNA sequences, such as promoters, polyadenylation signals, additional restriction enzyme sites, multiple cloning sites, other coding segments, and the like, such that their overall length may vary considerably. It is therefore contemplated that a nucleic acid fragment of almost any length may be employed. For example, illustrative DNA fragments with total lengths of about 1,000, 500, 400, 300, 200, 100, 50, 40, 30, 25 and 20 base pairs in length (including all intermediate lengths) are contemplated to be useful in the embodiments of the present invention.

Further provided are nucleic acids comprising a polynucleotide which is at least 80% identical to the nucleotide sequence of SEQ ID NO: 2 or SEQ ID NO: 4, wherein said polynucleotide encodes an antigen or antigenic fragment specific for *Babesia canis canis.* Preferably, the identity of the nucleotide sequences is at least 85%, or at least 90%. More preferably, the identity of the nucleotide sequences is at least 91%, at least 92%, at least 93%, at least 94%, or at least 95%. Most preferred are nucleic acids comprising (or consisting of) a polynucleotide which has at least 96%, at least 97%, at least 98%, or at least 99% identity to the nucleotide sequence of SEQ ID NO: 2 or 4. Preferably, the nucleic acid of the present invention comprising (or consisting of) a polynucleotide with a certain % identity encodes a polypeptide having the same antigenic specificity as the amino acid sequence set forth in SEQ ID NO: 1 or SEQ ID NO: 3. Hence, in a preferred embodiment, the nucleic acid of the present invention comprising (or consisting of) a polynucleotide with (at least) 80%, 85%, 90%, 91%, 92%, 93%, 94%, 95%, 96%, 97%, 98%, or 99% identity to the nucleotide sequence of SEQ ID NO: 2 or 4 encodes an antigen specific for *Babesia canis canis.*

Also provided are nucleic acids that are complementary to any of the nucleic acids described above.

As used herein, the terms "nucleic acid molecule", "nucleic acid" and "polynucleotide" are used interchangeably. Similarly, the terms "nucleic acid sequence" and "nucleotide sequence" are used interchangeably. A nucleic acid molecule of the present invention may be DNA or RNA, either single-stranded or double-stranded.

In another aspect, the present invention provides polypeptides encoded by the nucleic acids of the present invention. In one embodiment, the present invention provides polypeptides comprising an amino acid selected from the group consisting of the amino acid sequence shown in SEQ ID NO: 1 and SEQ ID NO: 3. In a preferred embodiment, the polypeptide has the amino acid sequence set forth in SEQ ID NO: 1 or SEQ ID NO: 3. Further provided are polypeptides having an amino acid sequence which is at least 80 % identical to the amino acid sequence of SEQ ID NO: 1 or SEQ ID NO: 3, or fragments thereof, wherein said polypeptide or fragment thereof is an antigen or antigenic fragment specific for *Babesia canis canis.*

As used herein, the terms "polypeptide", "peptide", and "protein" are used interchangeably. The term "recombinant polypeptide" refers to a polypeptide that is recombinantly expressed by a host cell via the use of a vector that has been modified by the introduction of a heterologous nucleic acid.

As used herein, the term "*Bcc*P33" refers to a polypeptide having the amino acid sequence as set forth in SEQ ID NO: 1. The corresponding nucleotide sequence encoding SEQ ID NO: 1 is set forth in SEQ ID NO: 2. The term "*Bcc*SA1" refers to a polypeptide having the amino acid sequence as set forth in SEQ ID NO: 3. The corresponding nucleotide sequence encoding SEQ ID NO: 3 is set forth in SEQ ID NO: 4. BccP33 and BccSA1 are polypeptides isolated from *Babesia canis canis* infected dogs. As shown experimentally, the BccP33 and *Bcc*SA1 polypeptides are *Babesia canis canis* specific antigens (i.e. antigens specific for *Babesia canis canis*). They do not cross-react with other *Babesia* species and can thus be used, for instance, to specifically detect antibodies against *Babesia canis canis.*

The present invention also relates to polypeptide variants that differ from the polypeptide having the amino acid sequence as set forth in SEQ ID NO: 1 or 3 in one or more substitution(s), deletion(s), insertion(s), and/or addition(s), provided that the antigenic specificity of the polypeptide is maintained, that is, the resulting polypeptide still is a *Babesia canis canis* specific antigen or antigenic fragment. Such variants may be identified by modifying one of the above amino acid sequences and evaluating the reactivity of the modified polypeptide with *Babesia canis canis*-specific antibodies or antisera, e.g. by using the methods described herein or by using any other suitable method known in the art. Amino acid substitutions are typically of single residues, but can occur at a number of different locations at once; deletions usually range in the order of 1 to 30 residues; and insertions usually are in the order of 1 to 10 amino acid residues. These variants are usually prepared by site-directed mutagenesis of nucleotides contained in the nucleotide sequence encoding the polypeptide, thereby producing a polynucleotide encoding the variant, and thereafter expressing the polynucleotide in recombinant cell culture. Deletions or insertions are preferably made in adjacent pairs, i.e. a deletion of 2 residues or insertion of 2 residues. Substitutions, deletions, insertions or any combination thereof may be combined to arrive at a final construct. The mutations must not place the sequence out of reading frame and preferably will not create complementary regions that could produce secondary mRNA structure. Preferably, substitutional variants are those in which at least one amino acid residue has been removed and a different amino acid residue inserted in its place such that a conservative substitution is obtained. The meaning of a conservative substitution is well known in the art. Additions include amino and/or carboxyl terminal fusions as well as intra-sequence insertions of single or multiple amino acid residues.

In a preferred embodiment, the polypeptide variant of the present invention comprises (or has) an amino acid sequence which has at least 80% identity to the amino acid sequence as set forth in SEQ ID NO: 1 or 3, or to a fragment of the amino acid sequence as set forth in SEQ ID NO: 1 or 3. Preferably, the identity of the amino acid sequence is at least 85%, or at least 90%. More preferably, the identity of the amino acid sequence is at least 91%, at least 92%, at least 93%, at least 94%, or at least 95%. Most preferred are polypeptide variants comprising (or having) an amino acid sequence which has at least 96%, at least 97%, at least 98% or at least 99% identity to the amino acid sequence of SEQ ID NO 1 or 3. Preferably, the described variants (which are variants of the BccP33 and BccSA1 polypeptides of the present invention) have the same antigenic specificity as BccP33 and BccSA1, respectively. Hence, in a preferred embodiment, the polypeptide with (at least) 80%, 85%, 90%, 91%, 92%, 93%, 94%, 95%, 96%, 97%, 98%, or 99% identity to the amino acid sequence as set forth in SEQ ID NO: 1 or SEQ ID NO: 3 is an antigen specific for *Babesia canis canis.*

Other preferred polypeptide variants include polypeptides comprising a fragment of the amino acid sequence as set forth in SEQ ID NO: 1 or 3, or a fragment having 80% identity to the amino acid sequence as set forth in SEQ ID NO: 1 or 3. As used herein, when referring to polypeptides, a "fragment" or "antigenic fragment" is a portion of a polypeptide/antigen of the present invention, wherein said portion is capable of reacting with a *Babesia canis canis*-specific antibody or with sera obtained from a *Babesia canis canis*-infected subject, as may be determined e.g. by using the ELISA assay described herein or any other suitable method known in the art. Polypeptides comprising at least an antigenic portion of one or more *Babesia canis canis* antigens as described herein may generally be used, alone or in combination, to detect *Babesia canis canis* in a subject.

Preferably, a fragment of the present invention comprises at least 10, at least 15, or at least 20 amino acids taken from the amino acid sequence of SEQ ID NO: 1 or 3. More preferably, the fragment comprises at least 30, at least 40 or at least 50 amino acids taken from the amino acid sequence of SEQ ID NO: 1 or 3. Most preferably, the polypeptide fragment of the present invention comprises at least 100, at least 150, or at least 200 amino acids of the amino acid sequence of SEQ ID NO: 1 or 3.

As used herein, in the context of two or more nucleic acids or polypeptide sequences, the terms "identical" or "having percent (%) identity" refer to two or more sequences that are the same ("identical") or have a specified percentage of nucleotides or amino acid residues that are the same, if compared and aligned for maximum correspondence. For sequence comparison, typically one sequence acts as a reference sequence, to which one or more test sequence(s) are compared. When using a sequence comparison algorithm, test and reference sequences are entered into a computer, subsequence coordinates are designated, if necessary, and sequence algorithm program parameters are designated. Default program parameters may be used, or alternative parameters can be designated. The sequence comparison algorithm then calculates the percent sequence identities for the test sequences relative to the reference sequence, based on the program parameters. Computer software that can be used for determining percent nucleotide or amino acid identity include those which is publically available, e.g. BLAST, BLAST-2, ALIGN or Megalign (DNASTAR) software. For example, a "comparison window" refers to a portion of at least 20 contiguous positions, preferably ate least 30, 40, 50, 60, or 70 positions.

In a preferred embodiment, the "percent (%) identity" is determined by comparing two optimally aligned sequences over a comparison window, wherein the portion of the polynucleotide or polypeptide sequence in the comparison window may comprise additions or deletions (*i.e*., gaps) as compared to the reference sequence (which does not comprise additions or deletions) for optimal alignment of the two sequences. The percentage is calculated by determining the number of positions at which the identical nucleotide or amino acid residue occurs in both sequences to yield the number of matched positions, dividing the number of matched positions by the total number of positions in the window of comparison and multiplying the result by 100 to yield the percentage of sequence identity.

In another aspect, the invention provides a vector comprising the nucleic acids of the present invention. In one embodiment, the vector of the present invention comprises a nucleic acid encoding a *Bcc*P33 polypeptide or a *Bcc*SA1 polypeptide, or a fragment thereof. Preferably, a nucleic acid comprised in the vector has the nucleotide sequence set forth in SEQ ID NO: 2 or SEQ ID NO: 4. Alternatively, the vector of the present invention may comprise a fragment of a nucleic acid encoding an amino acid sequence set forth in SEQ ID NO: 1 or SEQ ID NO: 3, preferably a fragment of the nucleotide sequence set forth in SEQ ID NO: 2 or SEQ ID NO: 4. As used herein, the term "vector" refers to a nucleic acid molecule used as a vehicle to transfer foreign genetic material into another cell. Vectors are typically composed of DNA, but RNA vectors are also comprised in the present invention. Vectors of the present invention include, but are not limited to, plasmids, viral vectors, cloning vectors and expression vectors. The expression constructs of the present invention may further contain sites for transcription initiation, termination and a ribosome binding site for translation. In various embodiments, the expression vectors of the present invention will include at least one selectable marker. Suitable selection markers are known to the skilled artisan.

In yet another aspect, the present invention provides a host cell comprising the nucleic acids of the present invention or the vector comprising any of said nucleic acids. As used herein, the term "host cell" refers to a cell that contains a vector and supports the replication and expression of the vector. Suitable host cells include prokaryotic cells such as *E. coli,* yeast and mammalian cells, such as the COS or CHO cell line. The polynucleotides expressed in this manner may encode the *Bcc*P33 and/or *Bcc*SA1 polypeptide, portions thereof or other variants thereof.

In a further aspect, the present invention provides methods of producing the polypeptides of the invention. Recombinant polypeptides may be readily prepared from a polynucleotide encoding the polypeptide using a variety of techniques well known in the art. Any of a variety of expression vectors known to those of ordinary skill in the art may be employed to express the polypeptides of the present invention. Expression may be achieved in any appropriate host cell that has been transformed or transfected with an expression vector containing polynucleotide that encodes a polypeptide of the present invention. In one embodiment, the present invention provides a method of producing the polypeptides of the invention comprising the steps of: (i) introducing a nucleic acid of the invention into a host cell, (ii) growing said host cell in a culture under suitable conditions to permit production of the polypeptide, and (iii) isolating the produced polypeptide. Alternatively, polypeptides of the present invention may be produced by a method comprising the following steps: (i) growing the host cell of the present invention in a culture under suitable conditions to permit production of the polypeptide, and (ii) isolating the produced polypeptide.

Recombinant proteins of the present invention can be isolated and purified from a host cell of the present invention containing or expressing the proteins/polypeptides by techniques known in the art including, but not limited to, lysis, chromatography, filtration, and centrifugation. In various embodiments, the proteins or polypeptides of the present invention, including, but not limited to, recombinant, isolated and/or purified proteins or polypeptides, are labeled. Preferably, the label is selected from the group consisting of an enzyme label, a radioisotope, a dye label, colloidal gold and biotin.

In another aspect, the present invention provides antibodies, or antigen binding fragments thereof that specifically bind to the polypeptides of the present invention. Preferably, the antibody or antigen binding fragment thereof specifically binds to a polypeptide comprising the amino acid sequence set forth in SEQ ID NO: 1 or SEQ ID NO: 3, or to a polypeptide consisting of the amino acid sequence set forth in SEQ ID NO: 1 or SEQ ID NO: 3, or to a polypeptide comprising a fragment of the amino acid sequence set forth in SEQ ID NO: 1 or SEQ ID NO: 3. The antibodies of the present invention may be used for detecting the polypeptides of the present invention in a sample. Hence, the antibodies of the present invention may be used for diagnosing a *Babesia canis canis* infection.

The antibody of the present invention may be selected from the group consisting of: a monoclonal antibody, a polyclonal antibody, a chimeric antibody, a Fab fragment, a F(ab')₂ fragment, and a scFv fragment. In various embodiments, the antibody of the present invention is labeled. Preferably, the label is selected from the group consisting of an enzyme label, a radioisotope, a fluorescent label, and biotin. The polypeptides of the present invention can be used to raise polyclonal and monoclonal antibodies provided by the present invention. The antibodies of the present invention may be prepared by any of a variety of methods available in the art and known to the skilled artisan.

The antibodies and antibody fragments provided by the present invention, whether attached to other sequences or not, can also include insertions, deletions, substitutions, or other selected modifications of particular regions or specific amino acids residues, provided the activity of the antibody or antigen binding fragment is not significantly altered or impaired compared to the non-modified antibody or antibody fragment. These modifications can provide for some additional property, such as to remove/add amino acids capable of disulfide bonding. In any case, antibodies or antigen binding fragments of the present invention must possess a bioactive property, such as specific binding to its cognate antigen. Functional or active regions of the antibodies or antigen binding fragments of the present invention may be identified by mutagenesis of a specific region of the protein, followed by expression and testing of the expressed polypeptide. Such methods are readily apparent to a skilled practitioner in the art and can include site-specific mutagenesis of the nucleic acid encoding the antibody or antibody fragment.

In another aspect, provided is a composition comprising a nucleic acid of the present invention, the vector of the present invention, a polypeptide of the present invention, and/or an antibody or antigen binding fragment of the present invention, or a mixture thereof. In certain embodiments, the composition is a pharmaceutical or diagnostic composition. In a preferred embodiment, the composition is a diagnostic composition and the component is selected from the following: (i) a nucleic acid comprising or consisting of a polynucleotide encoding a polypeptide having the amino acid sequence of SEQ ID NO: 1 or SEQ ID NO: 3 (preferably, a nucleic acid comprising or consisting of SEQ ID NO: 2 or 4); (ii) a polypeptide comprising or consisting of the amino acid sequence of SEQ ID NO: 1 or SEQ ID NO: 3; and (iii) an antibody or antigen-binding fragment thereof that specifically binds to a polypeptide comprising or consisting of the amino acid sequence of SEQ ID NO: 1 or SEQ ID NO: 3.

In another aspect, the present invention provides a vaccine comprising the nucleic acid of the present invention, the polypeptide of the present invention or an antigenic fragment thereof, and/or the vector of the present invention; or any mixture thereof. In one embodiment, the vaccine further comprises an adjuvant. An adjuvant in general is a substance that boosts the immune response of the target in a non-specific manner. Many different adjuvants are known in the art. Examples of adjuvants are Freund's complete and incomplete adjuvant, vitamin E, non-ionic block polymers and polyamines such as dextransulphate, carbopol and pyran. Also suitable are saponins. In a preferred embodiment, the vaccine of the present invention comprises cholera toxin as an adjuvant. The vaccine of the present invention may contain the nucleic acid either "naked" or in a delivery system, such as a bacterial or viral expression system.

Also contemplated is the use of the vaccines of the present invention, for instance in the methods described herein. In a preferred embodiment, the vaccine of the present invention is for use in preventing a *Babesia canis canis* infection, preferably in a dog or another *Canidae.*

In another aspect, the present invention provides a diagnostic kit comprising at least one of the components selected from: (i) the nucleic acid of the present invention; (ii) the polypeptide of the present invention, or an antigenic fragment thereof; (iii) the vector of the present invention; and (iv) the antibody of the present invention. In one embodiment, the kit is for detecting *Babesia canis canis* in a sample. In another embodiment, the kit is for diagnosing a *Babesia canis canis* infection in a *Canidae,* preferably a dog. Preferably, the kit provided by the present invention comprises a polypeptide of the present invention or an antigenic fragment thereof and is for diagnosing a *Babesia canis canis* infection in a dog by detecting antibodies against *Babesia canis canis* in a sample obtained from said dog. Alternatively, the kit comprises an antibody of the present invention, or an antigen binding fragment thereof, and is for diagnosing a *Babesia canis canis* infection in a dog by detecting a *Babesia canis canis* specific antigen in a sample obtained from said dog. Further alternatively, the kit comprises a nucleic acid of the present invention and is for diagnosing a *Babesia canis canis* infection in a dog by detecting a nucleic acid encoding a *Babesia canis canis* specific antigen or a fragment thereof in a sample obtained from said dog. In another aspect, the present invention provides a solid support comprising at least one polypeptide of the present invention. Alternatively, the solid support may comprise at least one nucleic acid of the present invention. The "solid support" may be any solid material known to those of ordinary skill in the art to which the polypeptide or nucleic acid may be attached. For example, the solid support may be a test well in a microtiter plate or a suitable membrane, such as nitrocellulose. Alternatively, the support may be a bead or a disc, such as glass, fiber glass, latex, or a plastic material such as polystyrene or polyvinyl chloride. The support may also be a magnetic particle. The polypeptides of the present invention may be immobilized to the solid support using a variety of techniques known in the art, including immobilization by direct or by indirect means. To bind a polypeptide to a solid support, both non-covalent association such as absorption, and covalent attachment may be used. The solid support may be used in the inventive methods of detecting antibodies against *Babesia canis canis* in a biological sample or diagnosing a *Babesia canis canis* infection in a subject.

In yet another aspect, the present invention provides a method of detecting antibodies against *Babesia canis canis* (that is, *B. canis canis* specific antibodies) in a biological sample. Preferably, the method comprises the steps of: (1) contacting the polypeptide of the present invention, or an antigenic fragment thereof, with the biological sample, and (2) detecting the presence of antibodies that bind to the polypeptide or antigenic fragment in the sample.

In a further aspect, the present invention provides a method of diagnosing a *Babesia canis canis* infection in a subject, comprising (1) contacting a polypeptide of the present invention, or an antigenic fragment thereof, with a sample obtained from a dog and (2) detecting the presence of antibodies (that is, *B. canis canis* specific antibodies) that bind to the polypeptide or antigenic fragment in the sample, wherein the presence of said detected antibodies is indicative of a *Babesia canis canis* infection. In a preferred embodiment, the subject infected with *Babesia canis canis* is a Canidae, preferably a dog.

Preferably, the step of detecting the presence of antibodies in the methods of the present invention is carried out by enzyme-linked immunosorbent assay (ELISA). In an ELISA assay, the step of contacting a polypeptide antigen with a biological sample may be performed by using a polypeptide that has been immobilized on a solid support, such as the well of a microtiter plate. In a first step, the polypeptide of the present invention is contacted with the biological sample under conditions that allow the binding of antibodies present in the biological sample to the polypeptide, so that antibody-antigen complexes are formed. In a second step, the formation of said antibody-antigen complexes is detected, wherein said detected antibody-antigen n complex is indicative of the presence of said antibodies against *Babesia canis canis.* Alternatively, the detection is carried out using other methods known in the art, such as ICT (immunochromatographic test), etc.

In the methods of the present invention, "cut-off levels" can be determined by using OD (optical density) values obtained with sera collected from non-infected animals, e.g. from SPF (specific pathogen free) dogs or from dogs in non-endemic areas.

In another aspect, the present invention provides the use of the nucleic acids, polypeptides, and/or antibodies of the present invention in the methods of the present invention. Particularly, provided is the use of a nucleic acid, a polypeptide, or an antibody of the present invention in a method of detecting *Babesia canis canis* and in a method of diagnosing a *Babesia canis canis* infection. In a preferred embodiment, the polypeptide of the present invention (or an antigenic fragment thereof), is used for detecting antibodies against *Babesia canis canis.* The polypeptides or antigenic fragment thereof, the nucleic acids, or the vector of the present invention may also be used for preparing a vaccine. Further provided is the use of a nucleic acid of the present invention, or a fragment thereof, as a probe, e.g. as a probe for detecting *Babesia canis canis* by hybridization.

As used herein, the term "biological sample" refers to any sample obtained from a subject, preferably a dog. In preferred embodiments, the sample is blood (e.g. whole blood, plasma, serum), or a blood component (e.g. erythrocytes). Other biological samples such as sputum, saliva, cerebrospinal fluid, synovial fluid, urine, or tissue may also be used.

In a preferred embodiment, an antibody of the present invention is used in a method for detecting the *Babesia canis canis*-specific polypeptides of the present invention, and the sample is blood erythrocytes.

The nucleic acids of the present invention may be used for detecting *Babesia canis canis,* e.g. in a biological sample such as blood or tissue, or for diagnosing a *Babesia canis canis* infection, e.g. in *Canidae* such as dogs. The detection or diagnosis of *Babesia canis canis* using the nucleic acids of the present invention is preferably carried out using a polymerase chain reaction (PCR). Other methods known in the art for detecting the nucleic acids encoding the polypeptides of the present invention, or their complements, are also contemplated. For example, fragments of the claimed nucleic acids, or their complements, may be used as probes for detecting nucleic acids encoding the polypeptides of the present invention, or their complements, by hybridization.

### EXAMPLES

The present inventors have constructed a cDNA expression library from *B. canis canis* and serologically screened the cDNA expression library. Two antigens were identified: a novel 33-kDa antigen and a secreted antigen. Their potential as candidates for serodiagnosis by ELISA was evaluated.

### 1. Parasites and sera

*B. canis canis* was maintained in liquid nitrogen. The splenectomized beagle infected *B. canis canis* was monitored by making Giemsa-stained thin blood smears. Blood was harvested with heparin when infected red blood cells reached or were near peak parasitemia (5-8%). Blood plasma was separated by centrifuging the harvested blood at 3,500 g for 15 min, and then kept at -80 °C.

Sera used for ELISA were as follows: 12 sera from dogs experimentally infected with *B. canis canis,* 3 sera from dogs experimentally infected with *B. canis vogeli*, 3 sera from dogs experimentally infected with *B. canis rossi*, 3 sera from dogs experimentally infected with *B. gibsoni*, 3 sera from dogs experimentally infected with *Neospora caninum*, and 28 sera from SPF dogs. In addition, 21 field serum samples were collected in the epidemic areas of Europe.

### 2. Construction of cDNA expression library

Total RNA was prepared from *B. canis canis*-infected splenectomized dog erythrocytes by the thiocyanate-phenol-chloroform extraction method. Then the poly(A)⁺ RNA was purified from the total RNA using oligotex-dT30 (JSR Co., Japan). Next, cDNA was synthesized by using a Zap-cDNA synthesis kit, ligated to a Uni-ZAP XR vector and packaged by using a Gigapack III packaging system according to the manufacturer's instructions (Stratagene, USA).

### 3. Immunoscreening of cDNA expression library.

The constructed library was plated on a total of 50 plates at a concentration of approximately 20,000 plaque-forming units (PFUs) per plate to lift plaques. The plaques were transferred to nitrocellulose membranes and screened with the serum prepared above according to the protocol of the picoBlue Immunoscreening Kit (Stratagene, USA). After an *in vivo* excision, the cDNA inserts in the positive clones were transferred into pBluescript phagemids and then sequenced with M13 forward, reverse, and internal DNA primers by using an automated sequencer (ABI PRISM 310 Genetic Analyzer, USA).

### 4. Expression and purification of recombinant BccP33 in E. coli.

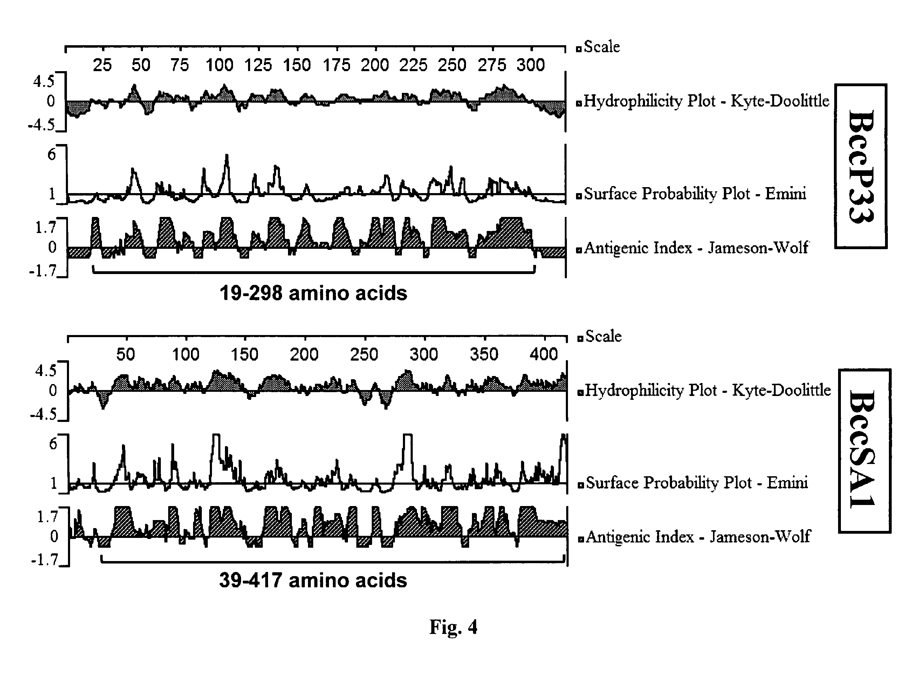

The entire fragment encoding *Bcc*P33 without its signal peptide and hydrophobic C-terminal region (amino acids 19-298), determined by software analysis (DNASTAR; NetWell Corporation, Tokyo, Japan) (Fig. 4), was amplified by PCR using primers with the introduced *Bam*HI and *Sal*I sites (underlined), P1 (5'-CGGGATCCGAAAACACTATACTTTTATCC -3') and P2 (5'- CGGTCGAC TTATTAAAGTTTAGGAGAAGCAGCAGT -3') and inserted into *Escherichia coli* expression vector pGEX-4T-1 (Amersham Pharmacia Biotech, USA). The resulting plasmid was designated as pGEX-4T-1/ *Bcc*P33 after it was identified by restriction enzyme analysis and sequencing. The recombinant protein fused with a glutathione S-transferase (GST) tag was expressed in the *E. coli* BL21 strain according to the manufacturer's instructions (Amersham Pharmacia Biotech, USA). Purification of recombinant *Bcc*P33 (r*Bcc*P33) was performed with glutathione-Sepharose 4B beads (Amersham Pharmacia Biotech, USA) according to the manufacturer's instructions.

### 5. Expression and purification of recombinant BccSA1 in E. coli

The entire fragment encoding *Bcc*SA1 without its signal peptide and hydrophobic N-terminal region (39-417 amino acids), determined by software analysis (DNASTAR; NetWell Corporation, Tokyo, Japan) (Fig. 4), was amplified by PCR using primers with the introduced *Eco*RI and *Sal*I sites (underlined), P1 (5'-CGGAATTCCAATCAACAAGCAGCCAG -3') and P2 (5'- CGGTCGAC CTAGTTGATTCATTCTTA -3'). Amplification was performed in a 50 µl of PCR buffer (Roche, Switzerland) containing 2.5 U of Taq polymerase, 10 pmol of each primer, 2 mM of each deoxynucleoside triphosphate, and 2 µl of DNA template. The PCR consisted of an initial denaturation at 96 °C for 5 min, followed by 30 amplification cycles (96 °C for 1 min, 58 °C for 1 min, and 72 °C for 90 s), and a final extension step at 72 °C for 7 min. The PCR product was cloned into *E. coli* expression vector pGEX-4T-1 (Amersham Pharmacia Biotech, USA). The resulting plasmid was identified by sequencing and designated as pGEX-4T-1/*Bcc*SA1. The recombinant *Bcc*SA1 was expressed as a glutathione S-transferase (GST) fusion protein in the *E*. *coli* BL21 strain according to the manufacturer's instructions (Amersham Pharmacia Biotech, USA). The resulting *E. coli* cells were washed with cold phosphate-buffered saline (PBS), lysed in a TNE buffer (50 mM Tris-HCl (pH 7.5), 100 mM NaCl, and 2 mM EDTA) containing 0.5% triton X-100 and 1 mg/ml lysozyme, sonicated, and then centrifuged at 15,000 g for 10 min at 4 °C. The supernatant containing soluble r*Bcc*SA1 was purified with glutathione-Sepharose 4B beads (Amersham Pharmacia Biotech, USA). Then the leader protein, GST, was cleaved by thrombin protease according to the manufacturer's instructions. The protein concentration was measured by using a modified Lowry protein assay kit (Thermo Scientific, USA).

### 6. Preparation of mouse anti-rBccP33 immune sera.

Five six-week-old female ICR mice (SLC, Japan) were intraperitoneally immunized with 100 µg of the purified r*Bcc*P33 emulsified with an equal volume of complete Freund's adjuvant (Difco Laboratories, USA). Two additional boosters with 50 µg of the rBccP33 antigen with incomplete Freund's adjuvant (Difco) were administered intraperitoneally at 2-week intervals. The mice were bled 14 days after the last booster, and serum samples were stored at -30°C.

### 7. Preparation of mouse sera against BccSA1.

Six-week-old female ICR mice (Clea, Japan) were immunized intraperitoneally with 100 µg of purified r*Bcc*SA1 without GST in an equal volume of Freund's complete adjuvant (Difco Laboratories, USA) for the first injection. One hundred micrograms of the same antigen in Freund's incomplete adjuvant (Difco) was injected intraperitoneally into the mice on days 14 and 28 post-primary injections. The mice were bled 14 days after the last booster, and serum samples were stored at -30°C.

### 8. Sodium dodecyl sulphate polyacrylamide gel electrophoresis (SDS-PAGE) and Western blotting for BccP33

To identify the native *BccP33* protein in the extract of *B. canis canis,* the *B. canis canis*-infected erythrocytes and normal erythrocytes were analyzed using SDS-PAGE and Western blot analysis as previously described. Briefly, the parasitized RBCs were harvested by low speed centrifugation (2,000 rpm, 10 min) and washed three times with PBS. The pellets were suspended in PBS containing saponin at a final concentration of 0.075% and incubated at 37°C in a water bath for 10 min to allow complete lysis of the RBC. The lysate was centrifuged at 8,000 rpm for 10 min at 4°C. Normal erythrocytes were treated similarly. The lysates of the *B. canis canis*-infected erythrocytes and normal erythrocytes were then sonicated and precipitated with acetone. The precipitated protein was dissolved in a sample buffer containing 5% 2-mercaptoethanol and boiled at 100°C for 10 min. Samples were electrophoresed on a 15% polyacrylamide gel before transferring to an Immobilon-P Transfer membrane. After blocking with 5% skim milk in PBS, the membrane was incubated with anti-rBccP33 antibody (1:100) at 37°C for 1 h. After washing with PBS containing 0.5% Tween 20, the membrane was soaked in peroxidase-conjugated goat anti-mouse immunoglobulin G (1:1,000) and incubated at 37°C for 1 h. Finally, the membrane was reacted with 3,3'-diaminobenzidine tetrahydrochloride and H₂O₂ to detect BccP33 protein.

### 9. Sodium dodecyl sulphate polyacrylamide gel electrophoresis (SDS-PAGE) and Western blotting for BccSA1

To identify the native *Bcc*SA1 in the parasite extract of *B*. *canis canis,* the *B. canis canis*-infected dog erythrocytes and normal hamster erythrocytes were analyzed by SDS-PAGE and Western blotting as described previously, with some modification. Briefly, the parasitized RBCs were harvested by centrifugation at 500 g for 10 min and washed three times with PBS. The pellets were treated with 0.075% of saponin in PBS (w/v) and incubated in a 37 °C water bath for 10 min to allow complete lysis of the RBCs. The lysate was centrifuged at 10,000 g for 10 min at 4°C. Normal erythrocytes were treated similarly. The lysates were then sonicated and precipitated with cold acetone. The precipitated protein was dissolved in SDS-PAGE sample buffer (125 mM Tris-HCl (pH 6.8), 4% (w/v) SDS, 20% (v/v) glycerol, 3% (v/v) 2-mercaptoethanol, and 0.02% bromophenol blue) and was boiled at 100°C for 5 min and then subjected to SDS-PAGE. After electrophoresis, the protein was transferred to an Immobilon-P transfer membrane (Millipore, USA). After being blocked with 5% skim milk in PBS overnight at 4°C, the membrane was incubated with mouse anti-r*Bcc*SA1 antibody (1:200) at room temperature for 2 hrs. After being washed with PBS containing 0.05% (v/v) Tween-20 (PBST), the membrane was soaked in peroxidase-conjugated goat anti-mouse immunoglobulin G (1:3,000) and incubated at room temperature for 1 hr. Finally, the membrane was reacted with 3, 3-diaminobenzidine tetrahydrochloride and H₂O₂ to detect *Bcc*SA1 protein. To determine the antibody response to *Bcc*SA1 in the dog infected with *B*. *canis canis,* r*Bcc*SA1 with or without GST or GST protein only was subjected to SDS-PAGE, transferred to a membrane, and probed with *B. canis canis*-infected dog serum.

### 10. Enzyme-linked immunosorbent assay (ELISA).

Purified r*Bcc* P33 fused GST, r*Bcc* SA1 fused GST or controls GST were diluted in a coating buffer (0.05 M carbonate-bicarbonate buffer, pH 9.6) to a final concentration of 4 µg/ml. Each well of 96-well microtiter plates (Nunc, Denmark) was coated with 100 µl of the protein overnight at 4°C. The subsequent protocols were performed as described previously. The cut-off value was defined as the mean value plus 3 standard deviations of the mean optical density (OD) obtained from 28 SPF dog serum samples.

### 11. Identification and characterization of the BccP33 gene

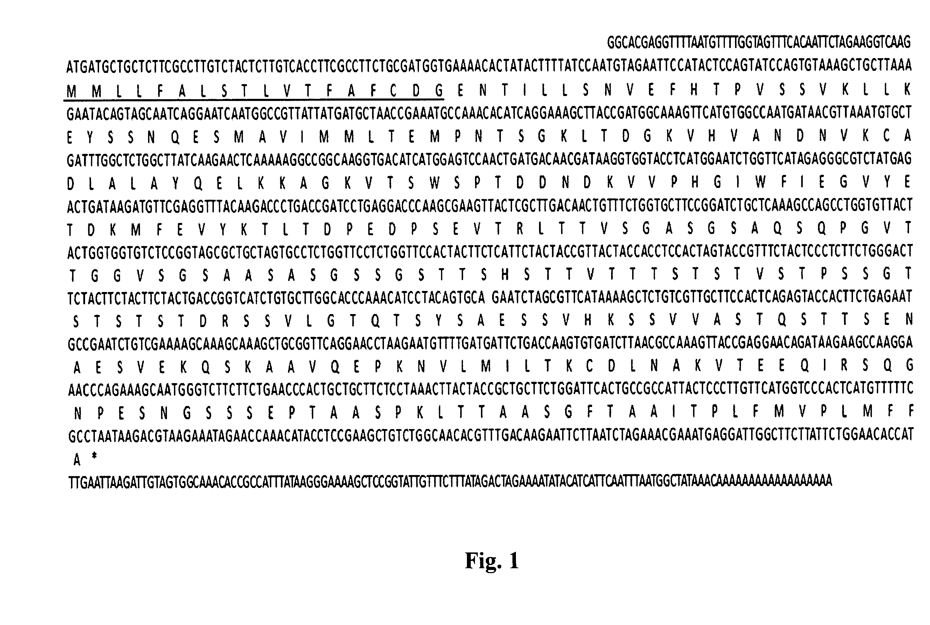

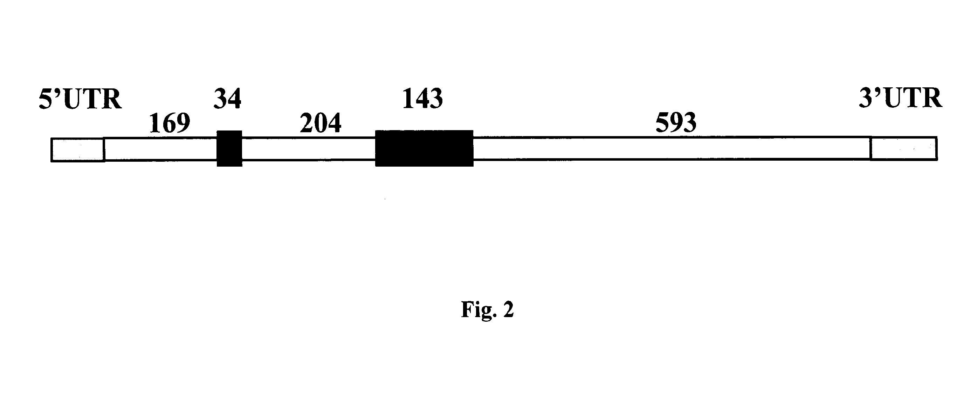

The full length of the *Bcc*P33 cDNA sequence had a single open reading frame of 966 nucleotides encoding a polypeptide of 321 amino acid residues. The molecular weight of the mature protein was 33.6 kDa, as calculated with software (SAPS, http://www.isrec.isb-sib.ch/software/SAPS_form.html/). Analysis of the putative N-terminal signal peptide in the *Bcc*P33 sequence by the SignalP server (http://www.cbs.dtu.dk/services/SignalP/) showed that this sequence had a high predicted signal peptide probability (0.999) and a maximum cleavage site probability (0.741) between amino acids in positions 18 and 19(Fig. 1). BLAST analysis of the amino acid sequence showed no significant similarity found in Genbank. In addition, *Bcc*P33 contained 2 introns (Fig. 2).

### 12. Identification and characterization of the BccSA1 gene

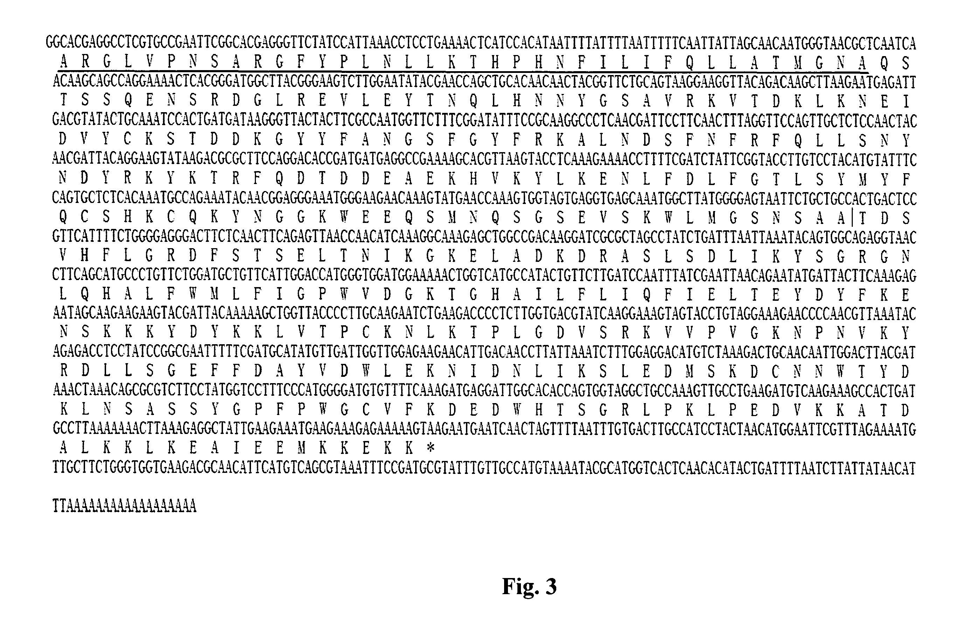

A total of 180 clones were obtained from the cDNA library with sera from the *B*. *canis*-infected dog. A novel gene named *Bcc*SA1 was identified from 37 clones. The partial length of *Bcc*SA1 contains 1,255 nucleotides encoding a polypeptide of 417 amino acid residues. Analysis of the putative N-terminal signal peptide in the *Bcc*SA1 sequence by the SignalP server (http://www.cbs.dtu.dk/services/SignalP/) showed that this sequence had a high predicted signal peptide probability (0.951) and a maximum cleavage site probability (0.512) between amino acids in positions 38 and 39. The mature protein consisted of 379 amino acid residues with a molecular weight of 44.1-kDa, as calculated by Statistical Analysis of Protein Sequences (SAPS, http://www.isrec.isb-sib.ch/software/SAPS form.html/). The amino acid sequence shared low homology with the *B.gibsoni* secreted antigen (*Bg*SA1) (Jia et al., 2010)(Fig. 3).

### 13. Expression of the BccP33 in E. coli

The *Bcc*P33 gene lacking the N-terminal signal peptide sequence was cloned into the prokaryotic expression vector pGEX-4T-1 and expressed in *E. coli* BL21 strain as a insoluble GST-fusion protein with a molecular weight of approximately 59-kDa including the 26-kDa GST tag. In addition, serum from a dog experimentally infected with *B. canis canis* recognized the r*Bcc*P33 GST-fusion protein in Western blotting, but there was no reaction with GST protein (Fig. 5).

### 14. Expression of the BccSA 1 in E. coli

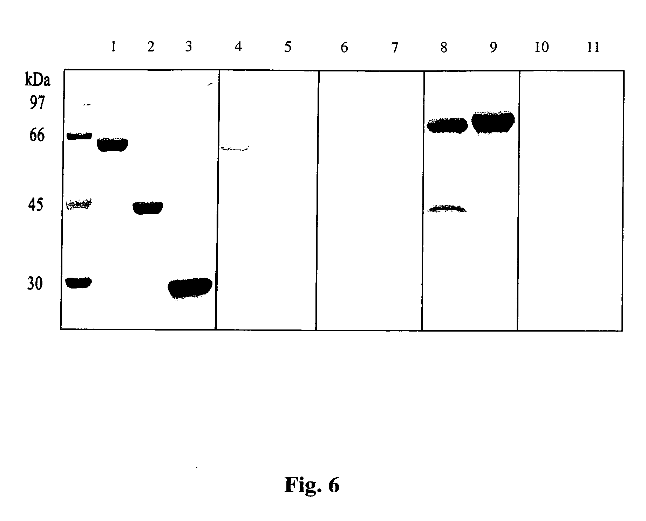

The *Bcc*SA1 lacking the N-terminal signal peptide sequence gene was cloned into the prokaryotic expression vector pGEX-4T-1, and the resulting plasmid was transformed into an *E. coli* BL21 strain. The rBccSA1 with a molecular weight of 66 kDa was expressed as a soluble GST-fusion protein with expected molecular weight. Sera from dogs experimentally infected with *B*. *canis canis* could recognize the GST-fused rBccSA1 in Western blotting. This result suggested that the r*Bcc*SA1 expressed in *E. coli* maintained their antigenicity. In addition, specific antibodies against *B. canis canis* were induced in both mice with immunization of r*Bcc*SA1 expressed in *E. coli* (Fig. 6).

### 15. Characterization of the native BccP33 of B. canis canis.

Mouse anti-r*Bcc*SA1 polyclonal sera were prepared and used to identify the native *Bcc*P33 in the *B. canis canis* parasites. The lysates of *B. canis canis*-infected and normal dog erythrocytes were analyzed by Western blotting. As shown in Fig. 5, a specific 33-kDa band was detected in *B. canis canis*-infected erythrocytes but not in normal dog erythrocyte lysate when probed with r*Bcc*P33-immunized mouse serum sample.

### 16. Characterization of the native BccSA1 of B. canis canis.

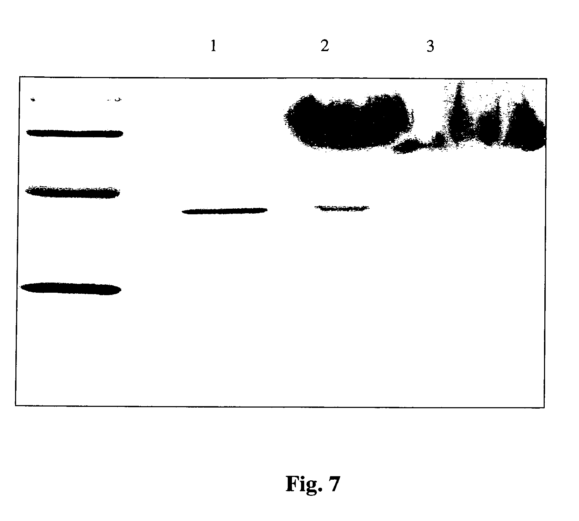

Mouse anti-r*Bcc*P33 polyclonal serum was used to identify the native *Bcc*SA1 in the lysate of *B*. *canis canis* parasites. As shown in Fig. 6, specific band with size of 44 kDa was detected in *B. canis canis*-infected red blood cells by Western blotting but not in normal ones. In addition, specific band was also detected in blood plasma with a parasitemia of 8% from a splenectomized dog experimentally infected with *B. canis canis* by using Western blotting (Fig. 7).

### 17. Application of recombinant BccP33 in an ELISA for the detection of a specific antibody.

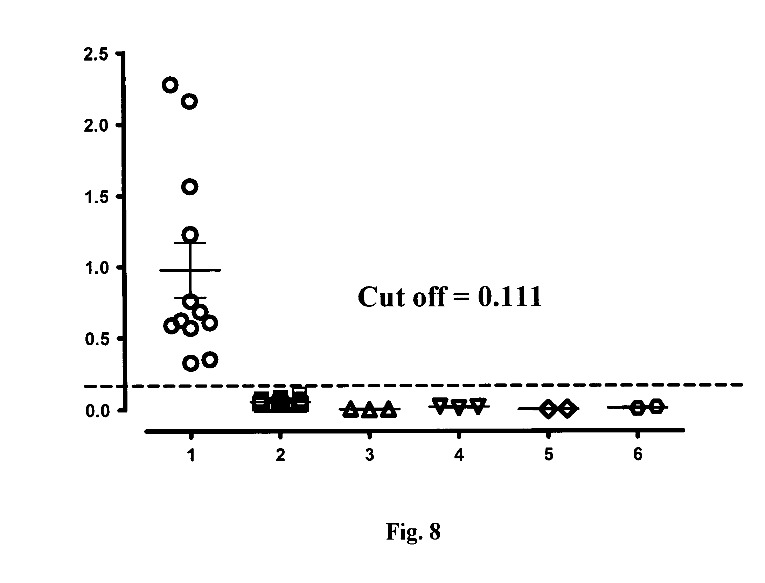

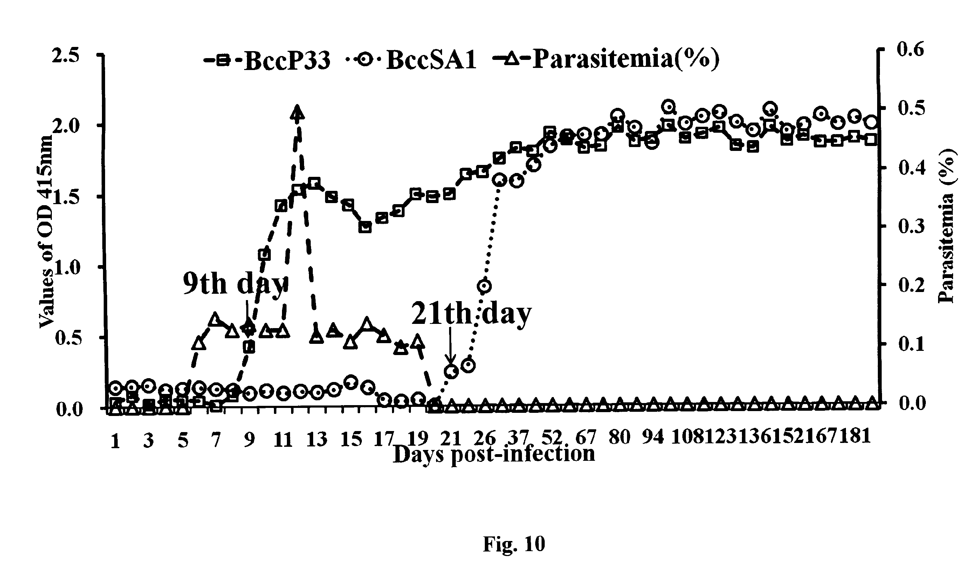

In order to validate the potential of r*Bcc*P33 as a diagnostic antigen, an indirect ELISA was performed to detect specific antibodies. As shown in Fig. 8, all 12 sera from dogs infected with *B.canis canis* were positive (lane 1, OD>0.111), whereas, all 28 sera from uninfected dogs were negative (lane 2, OD<0.111). 3 sera from *B*. *canis rossi*-infected dogs (lane 3), 3 sera from *B. canis vogeli*-infected dogs (lane 4), 3 sera from *B. gibson*i-infected dogs (lane 5) and *N. caninum*-infected dogs (lane 6) were negative (OD<0.024) At the same time, the specific antibodies against *Bcc*P33 were detectable as early as 9 days post-infection in *B. canis canis*-infected dog serum (Fig. 10). The antibody titers were maintained at a high level until 181 days post-infection, up to the chronic stage of infection in both strains, as evidenced by a recovering hematocrit value (data not shown) and a significantly low level of parasitemia.

### 18. Application of recombinant BccSA1 in an ELISA for the detection of a specific antibody.

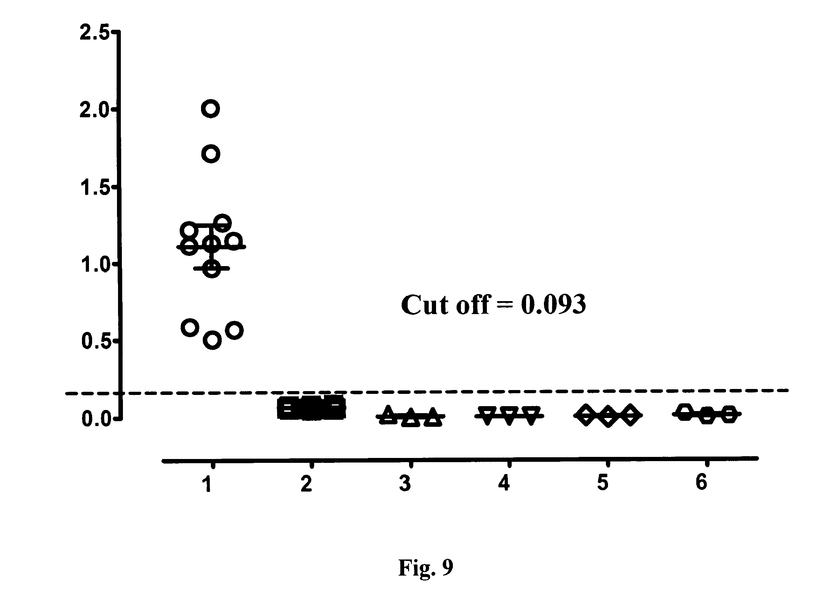

The potential of rBccSA1 as diagnostic antigen was evaluated in an indirect ELISA. All 11 serum samples from *B. canis canis*-infected dogs were positive (OD> 0.093), whereas the serum samples from the dogs infected with *B. canis rossi, B. canis vogeli, B. gibsoni*, *N. caninum* and uninfected dogs were negative (OD< 0.093) in the ELISA (Fig. 9). Specific antibody against *Bcc*SA1 could be detected on the 21^{st} day post-infection. The antibody level was maintained for 181 days post-infection even when the infection was in the chronic stage, which is characterized by a recovering hematocrit rate (data not shown) and a significantly low level of parasitemia (Fig. 10).

### 19. Detection of the specific antibody in field samples from Europe by ELISA.

A total of 21 dog serum samples from Europe were detected by ELISA with rBgSA1 and r*Bcc*P33 (see Table 1 below). 10 (47.6%) of the tested serum samples were positive in ELISA with r*Bcc*SA1, 13 (61.9%) of the tested samples were positive in ELISA with r*Bcc*P33. Moreover, all of the positive samples, except three in ELISA with r*Bcc*P33, were also positive in ELISA with r*Bcc*SA1 (see Table 2 below).

**Table 1. Comparison of BccSA1-ELISA with BccP33-ELISA for the detection of field samples from Europe.**

| No. (%) with *Bcc*SA1-ELISA | No. (%) with *Bcc*P33-ELISA | | Total no. (%) |
|---|---|---|---|
| | + | - | |
| + | 10(47.6) | 0(0) | 10(47.6) |
| - | 3(14.3) | 8(38.1) | 11(52.4) |
| Total no. (%) | 13(61.9) | 8(38.1) | 21(100) |

**Table 2. Value of BccSA1-ELISA and BccP33-ELISA for the detection of field samples from Europe.**

| Sample ID | Average value of *Bcc*P33 with GST | Average value of GST | Net value | Average value of *Bcc*SA1 with GST | Average value of GST | Net value | IFAT |
|---|---|---|---|---|---|---|---|
| 13.01925 | 0.264 | 0.171 | 0.093 | 0.214 | 0.196 | 0.018 | - |
| 12.01409 | 0.228 | 0.169 | 0.059 | 0.265 | 0.175 | 0.090 | - |
| 13.01423 | 0.445 | 0.148 | 0.298 | 0.314 | 0.162 | 0.153 | - |
| 30.01460 | 0.823 | 0.122 | 0.701 | 1.448 | 0.123 | 1.326 | + |
| 12.02134 | 0.501 | 0.164 | 0.338 | 0.437 | 0.178 | 0.259 | - |
| 10.02279 | 0.379 | 0.100 | 0.280 | 0.569 | 0.105 | 0.465 | + |
| 30.01715 | 0.221 | 0.127 | 0.094 | 0.167 | 0.086 | 0.081 | - |
| 09.02011 | 0.412 | 0.128 | 0.285 | 0.182 | 0.127 | 0.055 | + |
| 29.01353 | 0.399 | 0.143 | 0.256 | 0.174 | 0.149 | 0.025 | + |
| 1010750 o | 0.226 | 0.133 | 0.093 | 0.200 | 0.110 | 0.090 | - |
| 1010747 | 0.319 | 0.119 | 0.200 | 0.229 | 0.137 | 0.092 | + |
| 08/02012 | 1.949 | 0.124 | 1.826 | 1.043 | 0.124 | 0.919 | + |
| 1010749 | 0.644 | 0.099 | 0.545 | 0.382 | 0.170 | 0.212 | - |
| >1:256 o,1 | 0.715 | 0.097 | 0.618 | 1.604 | 0.119 | 1.486 | + |
| 1:128 | 0.227 | 0.119 | 0.108 | 0.314 | 0.134 | 0.180 | + |
| o o,1 | 0.271 | 0.165 | 0.106 | 0.292 | 0.202 | 0.090 | - |
| o o,2 | 0.187 | 0.090 | 0.097 | 0.111 | 0.081 | 0.030 | - |
| o o,3 | 0.419 | 0.366 | 0.053 | 0.367 | 0.294 | 0.074 | - |
| >1:256 o,2 | 0.376 | 0.121 | 0.255 | 1.188 | 0.555 | 0.634 | + |
| 1:256 o,1 | 0.756 | 0.165 | 0.591 | 0.630 | 0.167 | 0.463 | + |
| 1:256 o,2 | 0.458 | 0.134 | 0.324 | 0.216 | 0.128 | 0.088 | + |
| positive | 1.870 | 0.155 | 1.715 | 1.730 | 0.170 | 1.560 | + |
| negative | 0.153 | 0.091 | 0.062 | 0.136 | 0.096 | 0.040 | - |
| cut off | | | 0.111 | | | 0.093 | |

## Claims

1. Nucleic acid molecule selected from the group consisting of:
(a) a nucleic acid comprising a polynucleotide encoding a polypeptide having the amino acid sequence of SEQ ID NO: 1;
(b) a nucleic acid comprising a polynucleotide encoding a polypeptide having the amino acid sequence of SEQ ID NO: 3;
(c) a nucleic acid comprising the nucleotide sequence of SEQ ID NO: 2;
(d) a nucleic acid comprising the nucleotide sequence of SEQ ID NO: 4;
(e) a nucleic acid having the nucleotide sequence of SEQ ID NO: 2;
(f) a nucleic acid having the nucleotide sequence of SEQ ID NO: 4;
(g) a nucleic acid encoding a fragment of a polypeptide having the amino acid sequence of SEQ ID NO: 1 or SEQ ID NO: 3, wherein said polypeptide fragment is an antigenic fragment specific for *Babesia canis canis*;
(h) a nucleic acid comprising a polynucleotide which is at least 80% identical to the nucleotide sequence of SEQ ID NO: 2, wherein said polynucleotide encodes an antigen specific for *Babesia canis canis;*
(i) a nucleic acid comprising a polynucleotide which is at least 80% identical to the nucleotide sequence of SEQ ID NO: 4, wherein said polynucleotide encodes an antigen specific for *Babesia canis canis*;
(j) a nucleic acid which is at least 80% identical to the nucleic acid of (g), wherein the nucleic acid encodes a fragment of a polypeptide having the amino acid sequence of SEQ ID NO: 1 or SEQ ID NO: 3 and wherein said fragment is an antigenic fragment specific for *Babesia canis canis;* and
(k) a nucleic acid that is the complement of any of (a) to (j).

2. Polypeptide selected from the group consisting of:
(a) a polypeptide encoded by a nucleic acid molecule of any of claim 1 (a) to (j);
(b) a polypeptide comprising the amino acid sequence of SEQ ID NO: 1;
(c) a polypeptide comprising the amino acid sequence of SEQ ID NO: 3;
(d) a polypeptide having the amino acid sequence of SEQ ID NO: 1;
(e) a polypeptide having the amino acid sequence of SEQ ID NO: 3;
(f) a polypeptide having an amino acid sequence which is at least 80 % identical to the amino acid sequence of SEQ ID NO: 1, wherein said polypeptide is an antigen specific for *Babesia canis canis;*
(g) a polypeptide having an amino acid sequence which is at least 80 % identical to the amino acid sequence of SEQ ID NO: 3, wherein said polypeptide is an antigen specific for *Babesia canis canis;*
(h) a polypeptide comprising the polypeptide of (f) or (g); and
(i) a fragment of any of (a) to (g), wherein said polypeptide fragment is an antigenic fragment specific for *Babesia canis canis.*

3. Vector comprising a nucleic acid of claim 1.

4. Host cell comprising a nucleic acid of claim 1 or the vector of claim 3.

5. Antibody, or antigen-binding fragment thereof, that specifically binds to a polypeptide of claim 2.

6. Composition comprising at least one component selected from the group consisting of:
(i) a nucleic acid of claim 1;
(ii) a polypeptide of claim 2;
(iii) the vector of claim 3; and
(iv) the antibody or antigen-binding fragment thereof of claim 5.

7. Vaccine comprising at least one component selected from the group consisting of:
(i) a nucleic acid of claim 1;
(ii) a polypeptide of claim 2; and
(iii) the vector of claim 3.

8. Diagnostic kit comprising at least one component selected from the group consisting of:
(i) a nucleic acid of claim 1;
(ii) a polypeptide of claim 2;
(iii) the vector of claim 3; and
(iv) the antibody of claim 5.

9. Method of detecting antibodies against *Babesia canis canis* in a biological sample, comprising
(i) contacting a polypeptide of claim 2 with the biological sample, and
(ii) detecting the presence of antibodies that bind to the polypeptide or antigenic fragment in the sample.

10. Method of diagnosing a *Babesia canis canis* infection in a dog, comprising
(i) contacting a polypeptide of claim 2 with a biological sample obtained from the dog;
(ii) detecting the presence of antibodies that bind to the polypeptide or antigenic fragment in the sample, wherein the presence of said detected antibodies is indicative of a *Babesia canis canis* infection.

11. The method of claim 9 or 10, wherein the detection is carried out by ELISA.

12. Use of a nucleic acid of claim 1, or a polypeptide of claim 2, or the antibody of claim 5, in a method of detecting *Babesia canis canis.*

13. Use of a polypeptide of claim 2 in a method of detecting antibodies against *Babesia canis canis.*

14. Use of a nucleic acid of claim 1, or a polypeptide of claim 2, or the antibody of claim 5, in a method of diagnosing a *Babesia canis canis* infection.

15. Use of a nucleic acid of claim 1, or a polypeptide of claim 2, or the vector of claim 3, or the antibody of claim 5, for preparing a vaccine.
